# FASCICULE DE BREVET EUROPEEN

(11) **EP 0 711 530 B1**
(45) Date de publication et mention de la délivrance du brevet: **26.06.2002**
(21) Numéro de dépôt: 95402415.4
(22) Date de dépôt: 27.10.1995
(51) Int. Cl.: A61B 5/0424

(54) **Appareil, notamment de type Holter, pour l'enregistrement de signaux physiologiques, notamment d'activité cardiaque, comprenant un circuit de test de l'integrité des câbles d'électrode**
Gerät, insbesondere vom Typ Holter, zur Registrierung von physiologischen Signalen, insbesondere der Herzaktivität, mit einer Testschaltung der Integrität von Elektrodenkabeln
Apparatus, in particular of the Holter type, for recording physiological signals, especially the cardiac activity, comprising a circuit to test the integrity of the electrode cables

(30) Priorité: 28.10.1994 FR 9413008
(43) Date de publication de la demande: 15.05.1996
(73) Titulaire: ELA MEDICAL (Société anonyme), F-92541 Montrouge (FR)
(72) Inventeur: Faisandier, Yves, F-75014 Paris (FR)
(74) Mandataire: Dupuis-Latour, Dominique

(56) Documents cités:
- EP-A- 0 182 197
- EP-A- 0 335 977
- EP-A- 0 558 051
- US-A- 4 687 004
- US-A- 5 343 870

## Description

L'invention concerne un appareil, notamment de type Holter, pour l'enregistrement de signaux physiologiques, notamment d'activité cardiaque, recueillis par des électrodes externes appliquées à un patient.

On décrira essentiellement l'invention dans le cadre d'enregistrements ambulatoires de type "Holter", c'est-à-dire d'enregistrements, effectués en continu et sur une longue période, de signaux d'activité cardiaque recueillis au moyen d'électrodes externes (électrocardiogrammes) ; comme on le comprendra cependant, l'invention n'est pas limitée à ce type particulier d'enregistrements, et peut également concerner le recueil ou l'enregistrement d'autres données physiologiques telles que rythme respiratoire, tension artérielle, etc., dès lors que l'appareil de recueil et/ou d'enregistrement des signaux est relié à des électrodes distantes par l'intermédiaire de câbles.

De plus, l'appareil en question peut être aussi bien un appareil de type "Holter", c'est-à-dire un appareil porté par le patient, qu'un appareil installé à poste fixe.

Les câbles précités, qui sont soumis à de nombreuses contraintes, sont susceptibles de se couper d'une manière plus ou moins franche, conduisant à un recueil et un enregistrement défectueux des signaux physiologiques par l'appareil.

Cet aspect est particulièrement critique dans le cas des enregistrements Holter, dans la mesure où, d'une part, les différents éléments de l'appareil, et notamment les câbles, doivent être miniaturisés, et sont donc rendus plus fragiles, et où, d'autre part, ces câbles sont soumis à de nombreuses déformations, sollicitations, contraintes, etc.

Avant toute mise en route de l'enregistrement, il est donc absolument indispensable de s'assurer de l'intégrité des câbles et du bon état de leur connexion à l'enregistreur, d'une part, et à l'électrode de recueil, d'autre part.

Jusqu'à présent, ce test d'intégrité est réalisé soit à l'aide d'un ohmmètre sur lequel on branche les deux extrémités du câble - ce qui oblige à le débrancher - soit, après la mise en place des électrodes sur le patient et avant démarrage de l'enregistrement, par injection d'un courant de test sur chacun des câbles et mesure de la différence de potentiel résultante entre les deux bornes correspondant, respectivement, au câble testé et à l'électrode de masse (cf. EP-A-0 182 197, par exemple).

Si un défaut est ainsi détecté, l'appareil émet un message visuel ou sonore à l'intention de l'opérateur, afin de lui signaler le défaut. Ce système de scrutation cyclique permet à l'opérateur de s'assurer que les câbles sont tous intacts et les électrodes correctement appliquées en contact avec la peau, c'est-à-dire bien positionnées ; si tel n'est pas le cas, l'opérateur peut tenter de repositionner les électrodes jusqu'à obtenir un test d'intégrité positif.

Cependant, il n'existe pas de moyen permettant de discriminer sans ambiguïté entre un défaut provenant d'un mauvais positionnement d'une électrode et un défaut provenant d'une rupture du câble, si ce n'est pas tâtonnements successifs (l'impossibilité d'obtenir un test positif malgré un repositionnement de l'électrode laissant supposer que l'anomalie provient d'un défaut physique du câble et non d'un positionnement défectueux de l'électrode).

L'invention a pour objet de remédier à cet inconvénient, en proposant un moyen permettant, en cas de test négatif, de tester isolément et individuellement l'intégrité physique de chaque câble, et ce en laissant branché le câble à l'enregistreur.

L'appareil de l'invention est du type connu d'après le EP-A-0 182 197 précité, et comprenant : une pluralité de bornes de signal, propres à être reliées chacune à une extrémité proximale d'un câble dont l'extrémité distale est reliée à une électrode externe respective ; des moyens amplificateurs, recevant en entrée les signaux appliqués auxdites bornes de signal ; et des moyens de test de l'intégrité de l'ensemble des liaisons électriques au patient, ces moyens de test comprenant une source de courant de test, des moyens pour appliquer sur chacune des bornes ce courant de test et des moyens pour mesurer, par les moyens amplificateurs, l'impédance de la maille de circuit incluant le câble relié à la borne correspondante et dans laquelle circule le courant de test.

Selon l'invention, pour atteindre les buts précités, les moyens de test sont aptes à vérifier isolément l'intégrité physique desdits câbles (18) et comprennent à cet effet un plot (54) de test individuel de câble, ce plot étant disposé sur ledit appareil et étant relié à une source de courant, et pouvant être mis en contact avec l'extrémité distale (22) [d'un] dudit câble (18) désolidarisée de ladite électrode externe (24), l'extrémité proximale (20) de ce même câble (18) restant reliée à sa borne de signal (16) respective, le courant de test circulant (58) dans le câble entre cette borne (16) et le plot (54).

La source de courant reliée au plot peut être une source de courant continu ou bien alternatif, et peut en outre être la source de courant de test précitée.

De préférence, les moyens de test comprennent des moyens de mesure de la tension sur le plot et, avantageusement, des moyens pour mettre à la masse l'entrée des moyens amplificateurs reliés à la borne correspondant au câble à tester.

De préférence également, il est prévu un boîtier d'appareil comportant un couvercle mobile occultant, en position fermée, le plot de test individuel de câble ; avantageusement, en position fermée ce couvercle mobile occulte les bornes en empêchant toute désolidarisation des extrémités proximales des câbles d'avec ces bornes et/ou occulte un bouton d'initialisation de l'appareil et/ou verrouille en position un support de données extractible et/ou verrouille en position un connecteur de recueil de données complémentaires en provenance du patient.

D'autres caractéristiques et avantages de l'invention apparaîtront à la lecture de la description détaillée ci-dessous d'un exemple de réalisation, faite en référence aux dessins annexés.

La figure 1 est une vue générale d'un appareil de type Holter pouvant être utilisé dans le cadre de la présente invention.

La figure 2 est une représentation schématique des circuits électroniques de l'appareil de la figure 1, permettant la mise en oeuvre des principes de l'invention.

La figure 3 est une vue perspective partielle de la région postérieure de l'appareil, montrant divers organes susceptibles d'être occultés par la fermeture d'un couvercle spécifique.

La figure 4 est une vue perspective de trois-quarts arrière du connecteur de raccordement des électrodes à l'appareil.

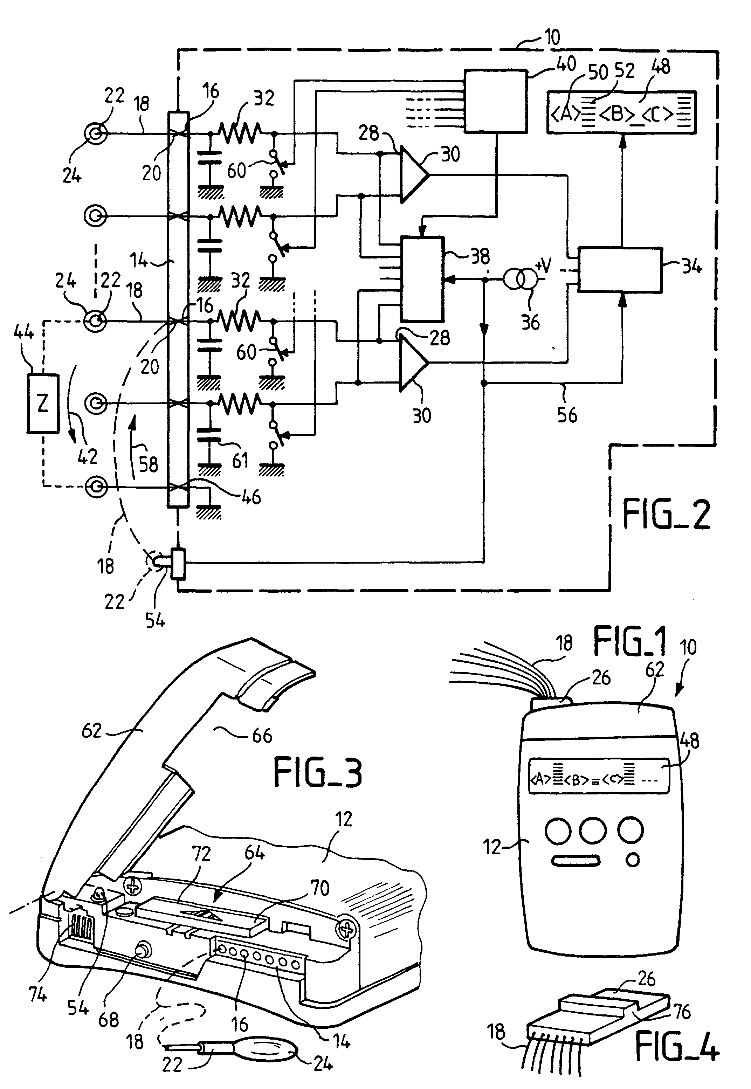

Sur la figure 1, l'appareil 10 comprend un boîtier 12 comportant un certain nombre de circuits, en eux-mêmes connus, permettant l'enregistrement des signaux physiologiques et la commande de l'appareil, et qui ne seront pas exposés en détail.

Comme on peut le voir sur le schéma de la figure 2, l'appareil 10 comporte un bornier 14 (visible également sur la vue perspective arrière de la figure 3) avec une pluralité de bornes 16, typiquement au nombre de sept dans le cas d'un appareil d'enregistrement des ECG, dont chacune est raccordée à un câble de liaison 18 par l'une des extrémités 20 de ce dernier. L'autre extrémité 22 de chaque câble 18 est connectée à une électrode respective 24 appliquée à un patient pour permettre le recueil des signaux physiologiques. Comme on peut le voir sur la figure 1, les divers câbles 18 peuvent être rassemblés en un faisceau relié au bornier 16 par l'intermédiaire d'un connecteur 26.

Chacune des bornes 16 est reliée à une entrée 28 d'un amplificateur 30 par l'intermédiaire d'une résistance de protection 32 ; les bornes 16 peuvent notamment, comme illustré, être raccordées aux amplificateurs 30 selon un montage différentiel, de manière en elle-même connue. Chacune des sorties des amplificateurs 30 est appliquée à un circuit de mesure 34 assurant le traitement des signaux recueillis et amplifiés (filtrage, numérisation, compression, enregistrement, affichage, etc.).

L'appareil comporte des moyens de test de l'intégrité des câbles, comprenant une source 36 de courant de test, courant qui est injectée successivement et de façon cyclique dans chacun des câbles 18 à travers la résistance série 32 et par l'intermédiaire d'un multiplexeur 38 commandé par un circuit logique de séquencement 40.

Le courant injecté dans le câble 18 va circuler, si ce câble n'est pas coupé, suivant la boucle de courant 42 au travers de l'impédance complexe 44 en retournant à l'appareil par une borne de masse 46 reliée à une électrode correspondante également appliquée au patient.

L'intensité du courant produit par la source 36 est de l'ordre de quelques microampères et ce courant peut être continu, alternatif ou, de préférence, impulsionnel (avec une durée d'impulsion de l'ordre de quelques dizaines de microsecondes, pour permettre la stabilisation des amplificateurs de mesure et la conversion du signal).

La circulation du courant injecté produit entre la borne concernée 16 et la masse une différence de potentiel qui est proportionnelle à la résistance du câble testé et du câble de retour de masse (quelques ohms si les câbles sont en bon état, quelques mégohms si l'un d'entre eux est défectueux), augmentée de celle du couple électrode-peau (à la fois au niveau testé et au niveau du retour de masse) et de l'impédance interne du corps du patient 44. Cette tension, amplifiée par le circuit 30, est d'autant plus faible que cette impédance d'ensemble est elle-même plus faible ; en pratique, cette impédance est soit très faible soit très élevée selon que, respectivement, le câble testé et le câble du retour de masse sont en bon état et les électrodes bien positionnées, ou non.

Avantageusement, le résultat des mesures successives est présenté sur l'afficheur 48 de l'appareil sous forme d'une indication 50 de canal (A, B, C, ...) et d'un graphique en barres 52, dont la hauteur sera d'autant plus faible que la tension sera élevée. L'opérateur est ainsi informé de la qualité de l'ensemble des connexions : lorsque toutes les barres 52 sont à leur hauteur maximum, la connexion est parfaite ; si une ou plusieurs d'entre elles sont faibles, il peut tenter de repositionner l'électrode correspondante pour voir s'il est possible de remédier au défaut ; si toutes les liaisons sont défectueuses, il s'agit d'un défaut de connexion ou de retour de masse, ce que le système peut déduire et indiquer par un message spécifique.

Comme on peut le voir, à ce stade il est seulement possible de dépister l'existence d'un défaut sur une ou plusieurs des connexions, mais sans pouvoir discriminer entre un mauvais positionnement d'électrode et un défaut d'intégrité physique du câble, si ce n'est pas tâtonnements en essayant de repositionner autrement l'électrode, ou encore en débranchant le faisceau de câbles et en le testant au moyen d'un ohmmètre ou autre contrôleur de continuité.

Pour pallier cette difficulté, l'invention propose de pourvoir l'appareil d'un dispositif de test individuel de câble permettant de vérifier isolément l'intégrité physique d'un câble tout en laissant celui-ci connecté à l'entrée de l'appareil.

Pour ce faire, l'appareil comporte un plot de test 54, par exemple en forme d'élément saillant métallique accessible de l'extérieur tel que celui représenté sur la figure 3. Ce plot est relié à une source de courant, avantageusement la source 36 déjà utilisée par ailleurs à l'étape précédente, et on mesure la tension sur le plot 54 ainsi alimenté en courant, par exemple au moyen de la ligne 56 aboutissant au circuit de mesure 34.

Si un défaut de ligne a été détecté de la manière indiquée ci-dessus, l'opérateur procède alors, dans un second temps, au test individuel du câble correspondant à cette ligne défectueuse.

Pour ce faire, il désolidarise l'extrémité 22 de l'électrode 24 et applique l'extrémité 22 du câble sur le plot de test 54.

Le courant produit par la source 36 va alors, si le câble n'est pas coupé, circuler suivant la maille 58 jusqu'à l'entrée 16 du bornier, puis dans la résistance 32 et retourner à la masse via un interrupteur 60 commandé par le circuit logique 40 (il s'agit par exemple d'interrupteurs statiques mettant à la masse les entrées des amplificateurs 30 pendant la durée de la phase du test individuel de câble). On notera que la résistance 32 permet d'éviter qu'en cas de grande surtension sur le câble (par exemple du fait d'une décharge d'électricité statique) le commutateur 60 ne soit détruit par la décharge et que, par la suite, un courant non négligeable ne s'écoule entre un point sous tension quelconque du circuit et le câble, conduisant à une électrolyse au niveau de l'électrode collée au patient.

En variante, la source de courant utilisée pour l'étape de test individuel de câble peut être une source de courant alternatif haute fréquence (typiquement de l'ordre du mégahertz). Dans ce cas, les condensateurs (préexistants) de filtrage des interférences radiofréquence tels que 61 court-circuitent à la masse les entrées des amplificateurs, jouant ainsi le même rôle que les commutateurs 60, dont on peut donc s'affranchir ainsi que leurs moyens de commande.

L'utilisateur peut être guidé dans sa manoeuvre par exemple au moyen de messages présentés sur l'afficheur 48. Ainsi, si le circuit de mesure 34 détecte un défaut de ligne au cours de la première étape de test, il peut afficher le message "CONNECTER CÂBLE X" (X étant la référence du câble de la liaison défectueuse). L'opérateur applique alors le câble sur le plot de test et, si l'impédance mesurée est faible, le message "CÂBLE OK" est présenté sur l'écran. En imprimant au câble des mouvements de traction et de flexion, l'opérateur vérifie que ce message reste bien affiché en permanence ; inversement si, même en imprimant au câble des mouvements de traction et de flexion, il n'arrive pas à afficher le message, ou si ce message n'est affiché que de façon intermittente suivant les déformations appliquées au câble, ceci indique sans ambiguïté que le défaut de ligne initialement détecté provient d'un défaut d'intégrité physique du câble et non d'un mauvais positionnement de l'électrode.

Très avantageusement, le plot de test 54 peut être disposé, comme illustré figure 3, à l'arrière de l'appareil et au voisinage de divers autres organes d'interfaçage ou organes accessibles de l'extérieur, l'ensemble étant fermé par un couvercle commun 62.

Ce couvercle 62, quand il est fermé, occulte la partie arrière 64 de l'appareil, l'évidement 66 permettant de laisser passer le connecteur 26 et les câbles 18 branchés sur le bornier 14.

Au début des opérations, après avoir disposé les électrodes sur le patient, l'opérateur ouvre le couvercle 62, relie les câbles 18 aux différentes bornes 16 du bornier 14 et met en route l'appareil par appui sur un bouton d'initialisation 68.

Il procède alors au dépistage préalable d'éventuels défauts de ligne, de la manière indiquée ci-dessus ; si un défaut est dépisté, il procède alors au test individuel du câble au moyen du plot 54.

Une fois ces opérations préalables achevées et si tout est en ordre, il ferme le couvercle 62, ce qui a pour effet de rendre inaccessible le plot de test 54 et le bouton d'initialisation 68, et également de verrouiller mécaniquement en place le connecteur 26 par l'évidement 66 coopérant avec un épaulement 76 (figure 4) du connecteur 26, empêchant ainsi toute séparation du faisceau de câbles d'avec l'appareil.

Avantageusement, ce couvercle peut également verrouiller en place une carte de mémoire statique 70 (notamment une carte de type "flash E²PROM") ou tout autre support extractible permettant l'enregistrement des données sur une longue durée dans laquelle seront enregistrés les signaux recueillis, cette carte 70 étant insérée dans un réceptacle 72 en partie arrière de l'appareil.

Le couvercle 62 peut également permettre de verrouiller mécaniquement non seulement le connecteur du faisceau de câbles relié aux électrodes mais également un connecteur de recueil de données complémentaires en provenance du patient, connecteur introduit dans une prise appropriée 74 située également à l'arrière de l'appareil.

## Revendications

1. Un appareil, notamment de type Holter, pour l'enregistrement de signaux physiologiques, notamment d'activité cardiaque, recueillis par des électrodes externes appliquées à un patient, cet appareil comprenant :
- une pluralité de bornes de signal (16), propres à être reliées chacune à une extrémité proximale (20) d'un câble (18) dont l'extrémité distale (22) est reliée à une électrode externe (24) respective,
- des moyens amplificateurs (30), recevant en entrée (28) les signaux appliqués auxdites bornes de signal, et
- des moyens de test de l'ensemble des liaisons électriques au patient, ces moyens de test comprenant une source de courant de test (36), des moyens (38, 40) pour appliquer sur chacune des bornes ce courant de test et des moyens (34) pour mesurer, par les moyens amplificateurs, l'impédance de la maille de circuit (42) incluant le câble relié à la borne correspondante et dans laquelle circule le courant de test,
appareil **caractérisé en ce que** les moyens de test sont aptes à vérifier isolément l'intégrité physique desdits câbles (18) et comprennent à cet effet un plot (54) de test individuel de câble, ce plot étant disposé sur ledit appareil et étant relié à une source de courant, et pouvant être mis en contact avec l'extrémité distale (22) [d'un] dudit câble (18) désolidarisée de ladite électrode externe (24), l'extrémité proximale (20) de ce même câble (18) restant reliée à sa borne de signal (16) respective, le courant de test circulant (58) dans le câble entre cette borne (16) et le plot (54).

2. L'appareil de la revendication 1, dans lequel la source de courant reliée au plot est une source de courant continu.

3. L'appareil de la revendication 1, dans lequel la source de courant reliée au plot est une source de courant alternatif.

4. L'appareil de la revendication 1, dans lequel la source de courant reliée au plot est ladite source de courant de test (36).

5. L'appareil de la revendication 1, dans lequel les moyens de test comprennent des moyens (34, 56) de mesure de la tension sur le plot.

6. L'appareil de la revendication 5, dans lequel les moyens de test comprennent en outre des moyens (40, 60) pour mettre à la masse l'entrée (28) des moyens amplificateurs (30) reliés à la borne correspondant au câble à tester.

7. L'appareil de la revendication 1, dans lequel il est prévu un boîtier d'appareil (12) comportant un couvercle mobile (62) occultant, en position fermée, le plot (54) de test individuel de câble.

8. L'appareil de la revendication 7, dans lequel, en position fermée, le couvercle mobile (62) occulte les bornes (14) en empêchant toute désolidarisation des extrémités proximales des câbles d'avec ces bornes.

9. L'appareil de la revendication 8, dans lequel, en position fermée, le couvercle mobile (62) occulte un bouton d'initialisation de l'appareil (68) et/ou verrouille en position un support de données extractible (70) et/ou verrouille en position un connecteur (74) de recueil de données complémentaires en provenance du patient.

## Patentansprüche

1. Vorrichtung, insbesondere vom Holter-Typ, für die Aufnahme physiologischer Signale, insbesondere einer Herzaktivität, die durch externe Elektroden empfangen werden, die an einen Patienten angelegt sind, wobei diese Vorrichtung aufweist:
- eine Mehrzahl von Signalanschlussklemmen (16), die geeignet sind, jeweils mit einem proximalen Ende (20) eines Kabels (18) verbunden zu werden, dessen distales Ende (22) mit einer jeweiligen äußeren Elektrode (24) verbunden ist,
- Verstärkermittel (30), die am Eingang (28) die Signale empfangen, die an die Signalanschlussklemmen angelegt sind, und
- Testmittel der Gesamtheit der elektrischen Verbindungen mit dem Patienten, wobei diese Testmittel eine Teststromquelle (36) aufweisen, Mittel (38, 40), um an jede dieser Anschlussklemmen diesen Teststrom anzulegen, und Mittel (34), um durch die Verstärkermittel die Impedanz der Schaltungsmasche (42) zu messen, einschließlich des Kabels, das mit der entsprechenden Anschlussklemme verbunden ist und bei welcher der Teststrom fließt,
wobei die Vorrichtung **dadurch gekennzeichnet ist, dass** die Testmittel dazu geeignet sind, isoliert die physische Unversehrtheit der Kabel (18) zu verifizieren und zu diesem Zweck eine Steckstelle für einen individuellen Kabeltest aufweisen, wobei diese Steckstelle auf der Vorrichtung angeordnet ist und mit einer Stromquelle verbunden ist und in Kontakt mit dem distalen Ende 22 des Kabels (18) gebracht werden kann, das von der äußeren Elektrode (24) getrennt ist, wobei das proximale Ende (20) dieses selben Kabels (18) mit seiner jeweiligen Signalanschlussklemme (16) verbunden bleibt, wobei der Teststrom (58) in dem Kabel zwischen dieser Anschlussklemme (16) und der Steckstelle (54) zirkuliert.

2. Vorrichtung nach Anspruch 1, bei welcher die Stromquelle, die mit der Steckstelle verbunden ist, eine Gleichstromquelle ist.

3. Vorrichtung nach Anspruch 1, bei welcher die Stromquelle, die mit der Steckstelle verbunden ist, eine Wechselstromquelle ist.

4. Vorrichtung nach Anspruch 1, bei welcher die Stromquelle, die mit der Steckstelle verbunden ist, die Teststromquelle (36) ist.

5. Vorrichtung nach Anspruch 1, bei welcher die Testmittel Messmittel (34, 56) der Spannung auf der Steckstelle aufweisen.

6. Vorrichtung nach Anspruch 5, bei welcher die Testmittel unter anderem Mittel (40, 60) aufweisen, um den Eingang (28) der Verstärkermittel (30), die mit der Anschlussklemme verbunden sind, entsprechend dem Kabel, das zu testen ist, an die Masse anzulegen.

7. Vorrichtung nach Anspruch 1, bei welcher ein Vorrichtungsgehäuse (12) vorgesehen ist, das einen bewegbaren Deckel (62) aufweist, der in geschlossener Position die Steckstelle (54) für einen individuellen Kabeltest bedeckt.

8. Vorrichtung nach Anspruch 7, bei welcher in geschlossener Position der bewegbare Deckel (62) die Anschlussklemmen (14) bedeckt, wobei jegliches Loslösen der proximalen Enden der Kabel mit diesen Anschlussklemmen verhindert wird.

9. Vorrichtung nach Anspruch 8, bei welcher in geschlossener Position der bewegbare Deckel (62) einen Startknopf der Vorrichtung (68) bedeckt und/oder in Position einen herausziehbaren Datenträger (70) und/oder in Position einen Stecker (74) für eine Aufnahme von komplementären Daten mit Herkunft vom Patienten verschließt.

## Claims

1. An appliance, in particular of the Holter type, for recording physiological signals, in particular heart activity, as picked up by external electrodes applied to a patient, the appliance comprising:
· a plurality of signal terminals (16) each suitable for being connected to a proximal end (20) of a respective cable (18) whose distal end (22) is connected to a respective external electrode (24);
· amplifier means (30) receiving as inputs (28) the signals applied to said signal terminals; and
· test means for testing the set of electrical connections with the patient, these test means comprising a test current source (36), means (38, 40) for applying said test current to each of the terminals, and means (34) for using the amplifier means to measure the impedance of the circuit loop (42) including the cable connected to the corresponding terminal and in which the test current flows,
the appliance being **characterized in that** the test means are suitable for verifying the physical integrity of said cables (18) in isolation and comprise, for this purpose, an individual cable test stud (54), said stud being placed on said appliance and being connected to a current source, and being capable of being put into contact with the distal end (22) of said cable (18) disconnected from said external electrode (24), the proximal end (20) of the same cable (18) remaining connected to its respective signal terminal (16), the test current flowing (58) in the cable between the terminal (16) and the stud (54).

2. The appliance of claim 1, in which the current source connected to the stud is a source of direct current.

3. The appliance of claim 1, in which the current source connected to the stud is a source of alternating current.

4. The appliance of claim 1, in which the current source connected to the stud is said test current source (36).

5. The appliance of claim 1, in which the test means comprise means (34, 56) for measuring the voltage at the stud.

6. The appliance of claim 5, in which the test means further comprise means (40, 60) for surrounding the input (28) of the amplifier means (30) connected to the terminal corresponding to the cable under test.

7. The appliance of claim 1, in which an appliance housing (12) is provided having a moving lid (62) which, in a closed position, hides the stud (54) for individual cable testing.

8. The appliance of claim 7, in which, in the closed position, the moving lid (62) hides the terminals (14) and prevents any disconnection between the proximal ends of the cables and the terminals.

9. The appliance of claim 8, in which, in the closed position, the moving lid (62) hides a button for initializing the appliance (68) and/or locks an extractable data medium (70) in position, and/or locks a connector (74) in position for picking up additional data coming from the patient.
